# EUROPEAN PATENT APPLICATION

(11) **EP 4 353 106 A1**
(43) Date of publication of application: **17.04.2024**
(21) Application number: 21960645.6
(22) Date of filing: 14.10.2021
(51) Int. Cl.: A24F 40/57

(54) **INHALATION DEVICE, BASE MATERIAL, AND CONTROL METHOD**

(71) Applicant: Japan Tobacco, Inc., Tokyo, 105-6927 (JP)
(72) Inventor: NAKAAE, Hiroki, Tokyo 130-8603 (JP)
(74) Representative: Hoffmann Eitle
(86) International application number: PCT/JP2021/038105
(87) International publication number: WO 2023/062788

(57) **Abstract**

[Problem] To provide a mechanism enabling further improvement of the quality of user experience pertaining to an inhalation device.

[Solution] An inhalation device comprising: a heating unit heating a base material containing an aerosol source to generate aerosol; and a control unit controlling operation of the heating unit on the basis of a temperature setting defining a time-series transition of a target temperature which is a target value of the temperature of the heating unit, the control unit controlling, on the basis of first puff information related to puff of the aerosol by a user in a first period among periods during which one base material is heated on the basis of the temperature setting, operation of the heating unit in a second period later than the first period.

## Description

### Technical Field

The present invention relates to an inhaler device, a substrate, and a control method.

### Background Art

Inhaler devices, such as e-cigarettes and nebulizers, that generate material to be inhaled by a user are widespread. For example, an inhaler device generates an aerosol having a flavor component imparted thereto, by using a substrate including an aerosol source for generating the aerosol, a flavor source for imparting the flavor component to the generated aerosol, and the like. A user is able to enjoy the flavor by inhaling the aerosol having the flavor component imparted thereto, which is generated by the inhaler device. An action of a user inhaling an aerosol will be hereinafter also referred to as a puff or a puff action.

Typically, an inhaler device generates an aerosol by heating a substrate. The quality of user experience is greatly affected by the temperature at which the substrate is heated, and thus techniques have been developed to achieve appropriate temperature control. For example, Patent Literature 1 below discloses a technique of controlling the temperature of heating in accordance with the type of substrate.

### Citation List

### Patent Literature

Patent Literature 1: JP 2012-513750 A

### Summary of Invention

### Technical Problem

However, in the technique disclosed in Patent Literature 1, the same temperature control is uniformly applied to any user, which makes it difficult to meet a user preference.

Accordingly, the present invention has been made in view of the above issue, and an object of the present invention is to provide a mechanism capable of further improving the quality of user experience regarding an inhaler device.

### Solution to Problem

To solve the above issue, according to an aspect of the present invention, there is provided an inhaler device including a heater configured to heat a substrate including an aerosol source to generate an aerosol; and a controller configured to control, based on a temperature setting defining a time-series transition of a target temperature which is a target value of a temperature of the heater, an operation of the heater. The controller is configured to control, based on first puff information regarding a puff of the aerosol taken by a user in a first period in a period during which the substrate which is a single substrate is heated based on the temperature setting, an operation of the heater in a second period subsequent to the first period.

The first puff information may include information indicating the number of puffs taken in the first period.

The controller may be configured to control, based on the first puff information, a temperature rising speed of the heater in the second period.

The controller may be configured to increase the temperature rising speed of the heater in the second period as the number of puffs indicated by the first puff information increases, and decrease the temperature rising speed of the heater in the second period as the number of puffs indicated by the first puff information decreases.

The controller may be configured to control, based on the first puff information, the target temperature in the second period.

The controller may be configured to increase the target temperature in the second period as the number of puffs indicated by the first puff information increases, and decrease the target temperature in the second period as the number of puffs indicated by the first puff information decreases.

The controller may be configured to control, based on the first puff information, a length of the second period.

The controller may be configured to decrease a length of the second period as the number of puffs indicated by the first puff information increases, and increase the length of the second period as the number of puffs indicated by the first puff information decreases.

The first puff information may include information indicating a change in a puff interval in the first period.

The controller may be configured to increase a temperature rising speed of the heater in the second period when the first puff information indicates that the puff interval becomes shorter with elapse of time, and decrease the temperature rising speed of the heater in the second period when the first puff information indicates that the puff interval becomes longer with elapse of time.

The controller may be configured to increase the target temperature in the second period when the first puff information indicates that the puff interval becomes shorter with elapse of time, and decrease the target temperature in the second period when the first puff information indicates that the puff interval becomes longer with elapse of time.

The controller may be configured to decrease a length of the second period when the first puff information indicates that the puff interval becomes shorter with elapse of time, and increase the length of the second period when the first puff information indicates that the puff interval becomes longer with elapse of time.

The controller may be configured to control, based on second puff information regarding a puff of the aerosol taken by the user in the second period, the operation of the heater in the second period.

The second puff information may include at least either one of information indicating the number of puffs taken in the second period or information indicating a change in a puff interval in the second period.

The controller may be configured to control, based on the second puff information, a temperature rising speed of the heater in the second period.

The controller may be configured to control, based on the second puff information, the target temperature in the second period.

The controller may be configured to control, based on the second puff information, a length of the second period.

The temperature setting may include an initial temperature rise period during which the temperature of the heater rises from an initial temperature, an intermediate temperature drop period during which the temperature of the heater drops after the initial temperature rise period, and a temperature re-rise period during which the temperature of the heater rises after the intermediate temperature drop period. The first period may be included in at least either one of the initial temperature rise period or the intermediate temperature drop period. The second period may be included in the temperature re-rise period.

To solve the above issue, according to another aspect of the present invention, there is provided a substrate that is to be heated by an inhaler device to generate an aerosol and that includes an aerosol source. The inhaler device includes a heater configured to heat the substrate including the aerosol source to generate an aerosol; and a controller configured to control, based on a temperature setting defining a time-series transition of a target temperature which is a target value of a temperature of the heater, an operation of the heater. The controller is configured to control, based on first puff information regarding a puff of the aerosol taken by a user in a first period in a period during which the substrate which is a single substrate is heated based on the temperature setting, an operation of the heater in a second period subsequent to the first period.

To solve the above issue, according to another aspect of the present invention, there is provided a control method for controlling an inhaler device including a heater configured to heat a substrate including an aerosol source to generate an aerosol. The control method includes controlling, based on a temperature setting defining a time-series transition of a target temperature which is a target value of a temperature of the heater, an operation of the heater. The controlling of an operation of the heater includes controlling, based on first puff information regarding a puff of the aerosol taken by a user in a first period in a period during which the substrate which is a single substrate is heated based on the temperature setting, an operation of the heater in a second period subsequent to the first period.

### Advantageous Effects of Invention

As described above, according to the present invention, there is provided a mechanism capable of further improving the quality of user experience regarding an inhaler device.

### Brief Description of Drawings

[Fig. 1] Fig. 1 is a schematic diagram of an inhaler device according to a configuration example.
[Fig. 2] Fig. 2 is a graph showing an example of the transition of the temperature of a heater when temperature control is performed based on the heating profile shown in Table 1.
[Fig. 3] Fig. 3 is a graph showing experimental results obtained by measuring the amount of flavor component delivered to a user per puff.
[Fig. 4] Fig. 4 is a graph showing experimental results obtained by measuring the amount of aerosol delivered to a user per puff.
[Fig. 5] Fig. 5 includes graphs each showing an example of puffs taken in a first period.
[Fig. 6] Fig. 6 is a graph showing an example of the transition of the temperature of the heater when temperature control based on first puff information according to the present embodiment is applied.
[Fig. 7] Fig. 7 is a flowchart illustrating an example of a flow of a process executed by the inhaler device according to the present embodiment.
[Fig. 8] Fig. 8 includes graphs each showing an example of puffs taken in a first period.

### Description of Embodiments

Hereinafter, a preferred embodiment of the present invention will be described in detail with reference to the accompanying drawings. In the specification and the drawings, structural elements having substantially the same functional configuration are denoted by the same reference signs, and a duplicate description will be omitted.

### <1. Configuration example>

An inhaler device generates material to be inhaled by a user. In the example described below, the material generated by the inhaler device is an aerosol. Alternatively, the material generated by the inhaler device may be gas.

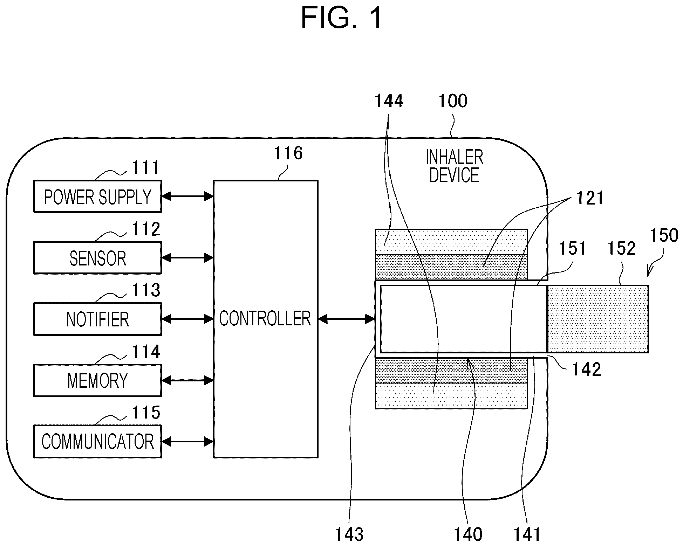

Fig. 1 is a schematic diagram of the inhaler device according to a configuration example. As illustrated in Fig. 1, an inhaler device 100 according to the present configuration example includes a power supply 111, a sensor 112, a notifier 113, a memory 114, a communicator 115, a controller 116, a heater 121, a holder 140, and a heat insulator 144.

The power supply 111 stores electric power. The power supply 111 supplies electric power to the structural elements of the inhaler device 100 under the control of the controller 116. The power supply 111 may be a rechargeable battery such as a lithium ion secondary battery.

The sensor 112 acquires various items of information regarding the inhaler device 100. In an example, the sensor 112 may be a pressure sensor such as a condenser microphone, a flow sensor, or a temperature sensor, and acquire a value generated in accordance with the user's inhalation. In another example, the sensor 112 may be an input device that receives information input by the user, such as a button or a switch.

The notifier 113 provides information to the user. The notifier 113 may be a light-emitting device that emits light, a display device that displays an image, a sound output device that outputs sound, or a vibration device that vibrates.

The memory 114 stores various items of information for operation of the inhaler device 100. The memory 114 may be a non-volatile storage medium such as flash memory.

The communicator 115 is a communication interface capable of communication in conformity with any wired or wireless communication standard. Such a communication standard may be, for example, Wi-Fi (registered trademark) or Bluetooth (registered trademark).

The controller 116 functions as an arithmetic processing unit and a control circuit, and controls the overall operations of the inhaler device 100 in accordance with various programs. The controller 116 includes an electronic circuit such as a central processing unit (CPU) or a microprocessor, for example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a flow path of air supplied to the stick substrate 150. An air inlet hole, which is an inlet of air to the flow path, is disposed in the bottom 143, for example. On the other hand, an air outlet hole, which is an outlet of air from the flow path, is the opening 142.

The stick substrate 150 includes a substrate 151 and an inhalation port 152. The substrate 151 includes an aerosol source. In the present configuration example, the aerosol source is not limited to a liquid, and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121 heats the aerosol source to atomize the aerosol source and generate the aerosol. In the example illustrated in Fig. 1, the heater 121 has a film-like shape and surrounds the outer circumference of the holder 140. Subsequently, heat produced from the heater 121 heats the substrate 151 of the stick substrate 150 from the outer circumference, generating the aerosol. The heater 121 produces heat when supplied with electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed so that the heater 121 protrudes from the bottom 143 of the holder 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed so that the heater 121 covers the bottom 143 of the holder 140. In still another example, the heater 121 may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may accommodate the stick substrate 150 while sandwiching the stick substrate 150 inserted into the internal space 141 by opening and closing the outer shell. In this case, the heater 121 may be at the sandwiching position of the holder 140 and may produce heat while pressing the stick substrate 150.

In addition, means for atomizing the aerosol source is not limited to heating by the heater 121. For example, the means for atomizing the aerosol source may be induction heating.

The inhaler device 100 and the stick substrate 150 cooperate with each other to generate an aerosol to be inhaled by the user. Thus, the combination of the inhaler device 100 and the stick substrate 150 may be regarded as an aerosol generation system.

### <2. Technical features>

### (1) Heating profile

The controller 116 controls the operation of the heater 121, based on a temperature setting. The control of the operation of the heater 121 is implemented by controlling supply of electric power from the power supply 111 to the heater 121. The temperature setting is information defining a time-series transition of a target temperature, which is a target value of the temperature of the heater 121. Hereinafter, such a temperature setting is also referred to as a heating profile.

The controller 116 controls the temperature of the heater 121 such that the transition of the temperature (hereinafter also referred to as an actual temperature) of the heater 121 becomes similar to the transition of the target temperature defined in the heating profile. The heating profile is typically designed to optimize the flavor that a user tastes when the user inhales an aerosol generated from the stick substrate 150. Thus, controlling of supply of electric power to the heater 121 based on the heating profile makes it possible to optimize the flavor that the user tastes.

The heating profile includes one or more combinations of a target temperature and information indicating a timing at which the target temperature is to be reached. The controller 116 controls the temperature of the heater 121 while switching the target temperature in accordance with the elapse of time from the start of heating based on the heating profile. Specifically, the controller 116 controls the temperature of the heater 121, based on the difference between a current actual temperature and a target temperature corresponding to the elapsed time from the start of heating based on the heating profile. The temperature control of the heater 121 can be implemented by, for example, known feedback control. The feedback control may be, for example, proportional-integral-differential controller (PID controller). The controller 116 may cause electric power from the power supply 111 to be supplied to the heater 121 in the form of pulses generated by pulse width modulation (PWM) or pulse frequency modulation (PFM). In this case, the controller 116 is capable of controlling the temperature of the heater 121 by adjusting the duty ratio or frequency of electric power pulses in the feedback control. Alternatively, the controller 116 may perform simple ON/OFF control in the feedback control. For example, the controller 116 may execute heating by the heater 121 until the actual temperature reaches the target temperature, stop heating by the heater 121 in response to the actual temperature reaching the target temperature, and execute heating by the heater 121 again in response to the actual temperature becoming lower than the target temperature. In addition, the controller 116 may adjust a voltage in the feedback control.

The temperature of the heater 121 can be quantified by, for example, measuring or estimating the electric resistance value of the heater 121 (more accurately, a heating resistor constituting the heater 121). This is because the electric resistance value of the heating resistor changes according to the temperature. The electric resistance value of the heating resistor can be estimated by, for example, measuring the amount of voltage drop in the heating resistor. The amount of voltage drop in the heating resistor can be measured by a voltage sensor that measures a potential difference applied to the heating resistor. In another example, the temperature of the heater 121 can be measured by a temperature sensor, such as a thermistor, installed near the heater 121.

A period from the start to the end of the process of generating an aerosol using the stick substrate 150 will be hereinafter also referred to as a heating session. In other words, the heating session is a period during which supply of electric power to the heater 121 is controlled based on the heating profile. The start of the heating session is a timing at which heating based on the heating profile is started. The end of the heating session is a timing at which a sufficient amount of aerosol is no longer generated. The heating session includes a first preheating period and a latter puffable period. The puffable period is a period during which a sufficient amount of aerosol is estimated to be generated. The preheating period is a period from the start of heating to the start of the puffable period. The heating performed in the preheating period is also referred to as preheating.

The heating profile may include a plurality of periods having, set therein, target temperatures different from each other. Temperature control may be performed such that a target temperature set in a certain period is reached at a certain timing in the period, or temperature control may be performed such that the target temperature is reached at the end of the period. In any case, it is possible to change the temperature of the heater 121 in a manner similar to the transition of the target temperature defined in the heating profile.

An example of the heating profile is shown in Table 1 below.

**[Table 1]**

| Table 1. Example of heating profile | | |
|---|---|---|
| Period | Elapsed time from start of heating | Target temperature |
| Initial temperature rise period | 0 sec. to 17 sec. | 310°C |
| | 17 sec. to 35 sec. | 310°C |
| Intermediate temperature drop period | 35 sec. to 45 sec. | 260°C |
| Temperature re-rise period | 45 sec. to 180 sec. | 290°C |
| | 180 sec. to 260 sec. | 290°C |
| Heating termination period | Thereafter | - |

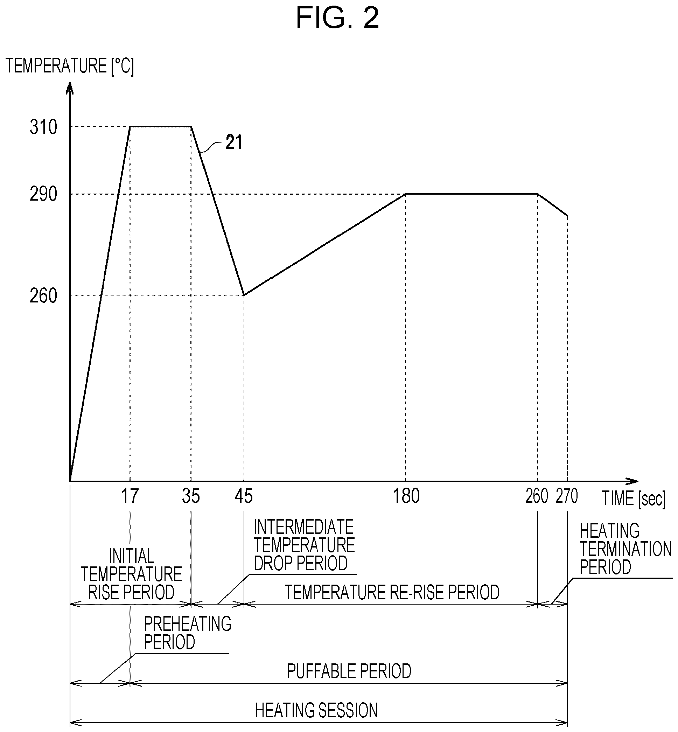

A description will be given of the transition of the temperature of the heater 121 in a case where the controller 116 performs temperature control in accordance with the heating profile shown in Table 1, with reference to Fig. 2. Fig. 2 is a graph showing an example of the transition of the temperature of the heater 121 in a case where temperature control is performed based on the heating profile shown in Table 1. The horizontal axis of this graph represents time (seconds). The vertical axis of this graph represents the temperature of the heater 121. The line 21 in this graph indicates the transition of the temperature of the heater 121. As illustrated in Fig. 2, the temperature of the heater 121 changes in a manner similar to the transition of the target temperature defined in the heating profile.

As shown in Table 1, the heating profile includes an initial temperature rise period at the beginning. The initial temperature rise period is a period during which the temperature of the heater 121 rises from an initial temperature. The initial temperature is the temperature of the heater 121 at the start of heating. As illustrated in Fig. 2, in the initial temperature rise period, the temperature of the heater 121 reaches 310°C after 17 seconds from the start of heating, and is maintained at 310°C until after 35 seconds from the start of heating. Accordingly, it is estimated that the temperature of the stick substrate 150 reaches a temperature at which a sufficient amount of aerosol is generated. Because the temperature rapidly rises to 310°C immediately after the start of heating, preheating can be finished early, and the puffable period can be started early. In Fig. 2, the preheating period ends after 17 seconds from the start of heating.

As shown in Table 1, the heating profile includes an intermediate temperature drop period that follows the initial temperature rise period. The intermediate temperature drop period is a period during which the temperature of the heater 121 drops. As illustrated in Fig. 2, in the intermediate temperature drop period, the temperature of the heater 121 drops from 310°C to 260°C from 35 seconds to 45 seconds after the start of heating. In this period, supply of electric power to the heater 121 may be stopped. Even in this case, a sufficient amount of aerosol is generated by the remaining heat of the heater 121 and the stick substrate 150. Here, if the heater 121 is maintained at a high temperature, the aerosol source included in the stick substrate 150 is rapidly consumed, which may cause deterioration of flavor, such as excessively strong flavor tasted by the user. In this regard, the intermediate temperature drop period provided in the middle makes it possible to avoid such deterioration of flavor and improve the quality of puff experience of the user.

As shown in Table 1, the heating profile includes a temperature re-rise period that follows the intermediate temperature drop period. The temperature re-rise period is a period during which the temperature of the heater 121 rises. As illustrated in Fig. 2, in the temperature re-rise period, the temperature of the heater 121 rises from 260°C to 290°C from 45 seconds to 180 seconds after the start of heating, and is maintained at 290°C until after 260 seconds from the start of heating. If the temperature of the heater 121 is continuously decreased, the temperature of the stick substrate 150 is also decreased. Thus, the amount of aerosol generated reduces, and the flavor tasted by the user may be deteriorated. In addition, as the heating profile progresses toward the end, the remaining amount of the aerosol source included in the stick substrate 150 decreases, and thus the amount of aerosol generated tends to reduce even if heating is continued at the same temperature. In this regard, re-rise of the temperature and an increase in the amount of aerosol generated in the latter half of the heating profile make it possible to compensate for a decrease in the amount of aerosol generated caused by a decrease in the remaining amount of the aerosol source. Accordingly, even in the latter half of the heating profile, it is possible to prevent deterioration of the flavor that the user tastes.

As shown in Table 1, the heating profile includes a heating termination period at the end. The heating termination period is a period that follows the temperature re-rise period, and is a period during which heating is not performed. The target temperature need not necessarily be set. As illustrated in Fig. 2, the temperature of the heater 121 decreases from 260 seconds after the start of heating. The supply of electric power to the heater 121 may be finished after 260 seconds from the start of heating. Even in this case, a sufficient amount of aerosol is generated for a while by the remaining heat of the heater 121 and the stick substrate 150. In the example illustrated in Fig. 2, the puffable period, that is, the heating session, ends after 270 seconds from the start of heating.

The user may be notified of the timing at which the puffable period starts and the timing at which the puffable period ends. Furthermore, the user may be notified of a timing that is a predetermined time before the end of the puffable period (for example, the timing at which supply of electric power to the heater 121 ends). In this case, the user is able to take a puff in the puffable period with reference to the notification.

### (2) Relationship between puff interval and quality of user experience

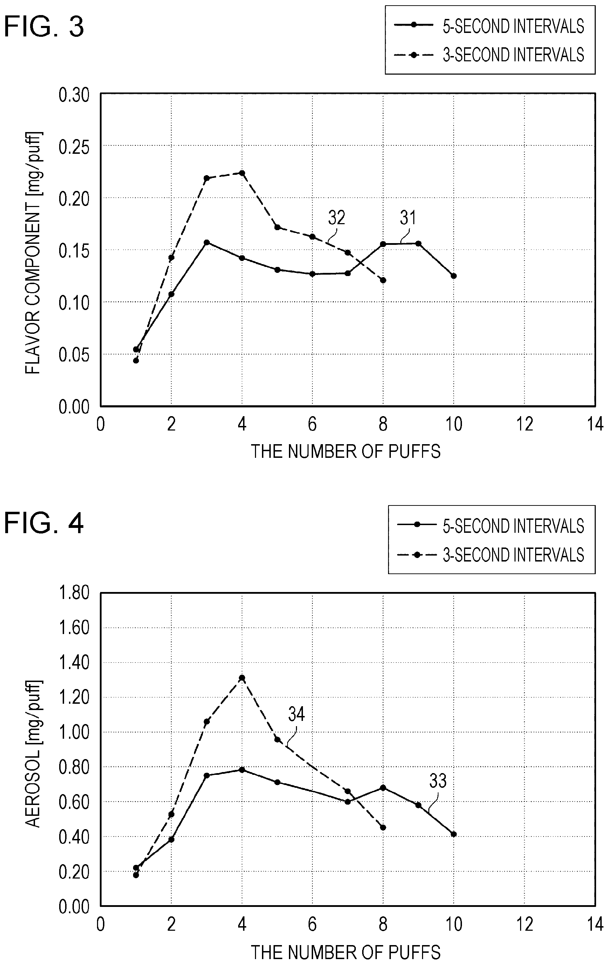

Hereinafter, the relationship between a puff interval and the quality of user experience will be described with reference to Figs. 3 and 4. A puff interval is an interval between puffs that are taken while the single stick substrate 150 is heated based on the heating profile. The number of puffs is the number of puffs that are taken while the single stick substrate 150 is heated based on the heating profile.

Fig. 3 is a graph showing experimental results obtained by measuring the amount of flavor component delivered to a user per puff. The line 31 indicates, for each number of puffs, the amount of flavor component delivered per puff when puffs are taken at 5-second intervals immediately after the end of preheating. The line 32 indicates, for each number of puffs, the amount of flavor component delivered per puff when puffs are taken at 3-second intervals immediately after the end of preheating. In these experiments, the stick substrate 150 is heated in accordance with the same heating profile.

Fig. 4 is a graph showing experimental results obtained by measuring the amount of aerosol delivered to a user per puff. The line 33 indicates, for each number of puffs, the amount of aerosol delivered per puff when puffs are taken at 5-second intervals immediately after the end of preheating. The line 34 indicates, for each number of puffs, the amount of aerosol delivered per puff when puffs are taken at 3-second intervals immediately after the end of preheating. In these experiments, the stick substrate 150 is heated in accordance with the same heating profile.

Referring to Figs. 3 and 4, when the puff interval is short, the amounts of flavor component and aerosol delivered per puff are large in an initial stage, and significantly decrease as the number of puffs increases. On the other hand, when the puff interval is long, the amounts of flavor component and aerosol delivered per puff are smaller than those when the puff interval is short, but are less likely to decrease even when the number of puffs increases.

Accordingly, it can be seen that the puff interval has a large influence on the amounts of flavor component and aerosol delivered to the user. A user having a short puff interval is estimated to have a preference of short-term concentration in which the user wants to inhale large amounts of flavor component and aerosol in a short period of time. On the other hand, a user having a long puff interval is estimated to have a preference of long-term stability in which the user wants to inhale stable amounts of flavor component and aerosol over a long period of time.

Thus, in the present embodiment, the operation of the heater 121 is controlled in accordance with the puff interval of the user, and thereby an optimal user experience that meets the preference of the user is provided.

### (3) Control based on first puff information

The controller 116 controls, based on first puff information regarding a puff of an aerosol taken by a user in a first period in a period during which the single stick substrate 150 is heated based on the heating profile, the operation of the heater 121 in a second period subsequent to the first period. The control of the operation of the heater 121 in the second period may be implemented by controlling the heating profile. That is, the controller 116 controls, based on the first puff information, the target temperature in the second period and/or the timing at which the target temperature is to be reached in the heating profile. With this configuration, when the user continuously takes puffs while using the single stick substrate 150, heating in a latter half is controlled in accordance with the tendency of puffs in a former half. Thus, while the user uses the single stick substrate 150, the preference of the user can be observed, and a puff experience that meets the observed preference of the user can be provided.

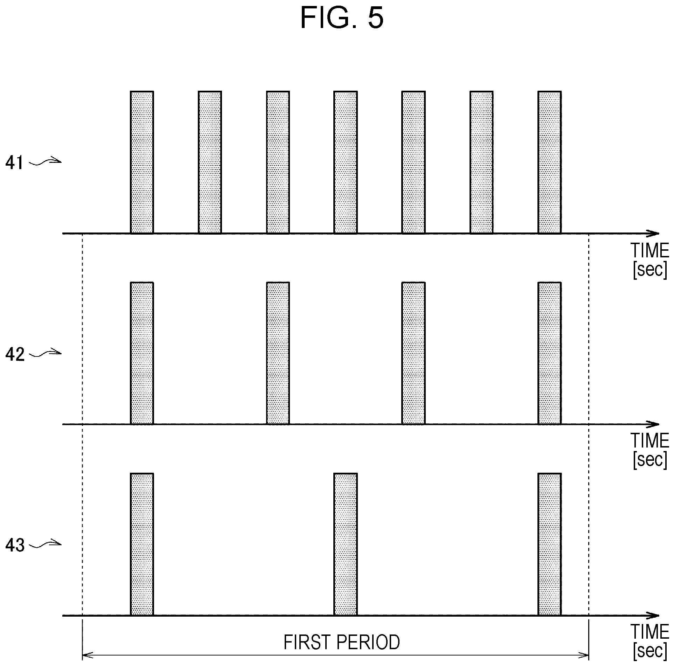

The first puff information may include information indicating the number of puffs taken in the first period. The first puff information may be the number of puffs per unit time taken in the first period. The number of puffs per unit time corresponds to a puff interval. Alternatively, the first puff information may be the number of puffs taken in the first period. When the length of the first period has a fixed value, the number of puffs taken in the first period corresponds to the puff interval in the first period. This configuration makes it possible to provide, in the second period, a user experience that meets the preference of the user estimated from the puff interval in the first period. The first puff information will be described in detail with reference to Fig. 5.

Fig. 5 includes graphs each showing an example of puffs taken in the first period. In graphs 41 to 43, the horizontal axis represents time, and a rectangle represents a timing at which a puff is taken. The graph 41 shows an example in which the number of puffs taken in the first period is large, that is, an example in which the puff interval is short. The graph 42 shows an example in which the number puffs taken in the first period is medium, that is, an example in which the puff interval is medium. The graph 43 shows an example in which the number of puffs taken in the first period is small, that is, an example in which the puff interval is long. As shown in the graph 41, a user having a large number of puffs and a short puff interval is estimated to have a preference of short-term concentration. On the other hand, as shown in the graph 43, a user having a small number of puffs and a long puff interval is estimated to have a preference of long-term stability.

The first period may be included in at least either one of the initial temperature rise period or the intermediate temperature drop period. The second period may be included in the temperature re-rise period. This configuration makes it possible to provide a user experience that meets the preference of the user in the latter half of the heating profile in which the amounts of flavor component and aerosol delivered per puff decrease, as illustrated in Figs. 3 and 4. The end of the first period and the start of the second period may coincide with each other or may be spaced apart from each other.

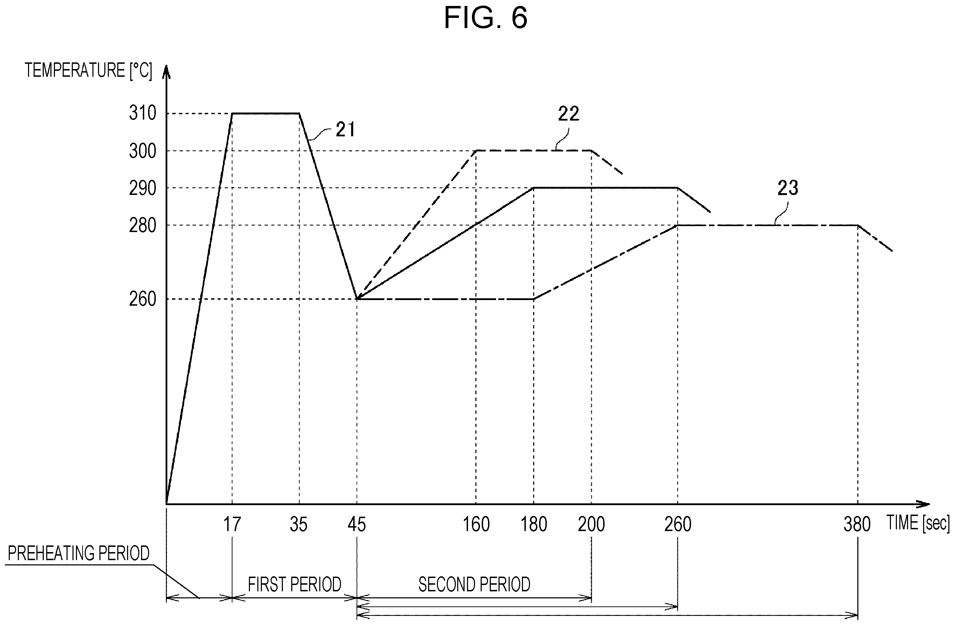

Hereinafter, an example of temperature control based on the first puff information will be specifically described with reference to Fig. 6. Fig. 6 is a graph showing an example of the transition of the temperature of the heater 121 when temperature control based on the first puff information according to the present embodiment is applied. The horizontal axis of this graph represents time (seconds). The vertical axis of this graph represents the temperature of the heater 121. In this example, the period that follows the preheating period in the initial temperature rise period and the entire intermediate temperature drop period correspond to the first period. The entire temperature re-rise period is the second period.

The line 21 in this graph indicates the transition of the temperature of the heater 121 when default temperature control, which is applied when the puff interval is medium, is applied. The default temperature control is applied when the number of puffs taken in the first period is equal to or greater than a first threshold value and smaller than a second threshold value. The first threshold value is smaller than the second threshold value. For example, the first threshold value is four, and the second threshold value is seven. When the default temperature control is applied, the temperature of the heater 121 changes in a manner similar to the transition of the target temperature defined in the heating profile shown in Table 1. According to the line 21, the temperature of the heater 121 rises from 260°C to 290°C from 45 seconds to 180 seconds after the start of heating. Thereafter, the temperature of the heater 121 is maintained at 290°C for 80 seconds until after 260 seconds from the start of heating.

The line 22 in this graph indicates the transition of the temperature of the heater 121 when the temperature control that is applied when the puff interval is short is applied. The temperature control that is applied when the puff interval is short is applied when the number of puffs taken in the first period is equal to or greater than the second threshold value. According to the line 22, the temperature of the heater 121 rises from 260°C to 300°C from 45 seconds to 160 seconds after the start of heating. Thereafter, the temperature of the heater 121 is maintained at 300°C for 40 seconds until after 200 seconds from the start of heating.

The line 23 in this graph indicates the transition of the temperature of the heater 121 when the temperature control that is applied when the puff interval is long is applied. The temperature control that is applied when the puff interval is long is applied when the number of puffs taken in the first period is smaller than the first threshold value. According to the line 23, the temperature of the heater 121 is maintained at 260°C from 45 seconds to 180 seconds after the start of heating. Thereafter, the temperature of the heater 121 rises to 280°C until after 260 seconds from the start of heating. Thereafter, the temperature of the heater 121 is maintained at 280°C for 120 seconds until after 380 seconds from the start of heating.

As illustrated in Fig. 6, the controller 116 controls, based on the first puff information, the temperature rising speed of the heater 121 in the second period. In particular, the controller 116 controls, based on the first puff information, the temperature rising speed from when the second period is started to when the maximum target temperature in the second period (hereinafter, also referred to as a second target temperature) is reached. The temperature rising speed in the line 21 is expressed by (290°C - 260°C)/(180 seconds - 45 seconds) ≈ 0.22°C/second. The temperature rising speed in the line 22 is expressed by (300°C - 260°C)/(160 seconds - 45 seconds) ≈ 0.35°C/second. The temperature rising speed in the line 23 is expressed by (280°C - 260°C)/(260 seconds - 45 seconds) ≈ 0.09°C/second. As a result of controlling the temperature rising speed in the second period, the amounts of aerosol and flavor component delivered to the user immediately after the start of the second period can be controlled. Accordingly, it is possible to provide a user experience that meets the preference of the user.

Specifically, as indicated by the line 22, the controller 116 increases the temperature rising speed of the heater 121 in the second period as the number of puffs indicated by the first puff information increases. As a result of increasing the temperature rising speed, the actual temperature of the heater 121 can be increased early. This configuration makes it possible to compensate for a decrease in the delivery amounts of flavor component and aerosol caused by an increase in the number of puffs that occurs when the puff interval is short, described above with reference to Figs. 3 and 4, by increasing the delivery amounts of flavor component and aerosol in accordance with a rapid temperature rise. That is, it is possible to improve the quality of user experience provided to a user who is estimated to have a preference of short-term concentration.

On the other hand, as indicated by the line 23, the controller 116 decreases the temperature rising speed of the heater 121 in the second period as the number of puffs indicated by the first puff information decreases. As a result of decreasing the temperature rising speed, the actual temperature of the heater 121 can be increased slowly. As described with reference to Figs. 3 and 4, when the puff interval is long, the delivery amounts of flavor component and aerosol are less likely to decrease, and thus the life of the stick substrate 150 (that is, the period until the aerosol source is exhausted) can be extended by decreasing the temperature rising speed. That is, it is possible to improve the quality of user experience provided to a user who is estimated to have a preference of long-term stability.

As illustrated in Fig. 6, the controller 116 controls, based on the first puff information, the target temperature in the second period. In particular, the controller 116 controls the second target temperature based on the first puff information. The second target temperature in the line 21 is 290°C. The second target temperature in the line 22 is 300°C. The second target temperature in the line 23 is 280°C. As a result of controlling the target temperature in the second period, the amounts of aerosol and flavor component delivered to the user in the second period, and the length of the puffable period, can be controlled. Accordingly, it is possible to provide a user experience that meets the preference of the user.

Specifically, as indicated by the line 22, the controller 116 increases the target temperature in the second period as the number of puffs indicated by the first puff information increases. It is estimated that as the target temperature increases, the amounts of aerosol and flavor component delivered to the user per puff increase, and the life of the stick substrate 150 decreases. In this regard, this configuration makes it possible to provide an optimal user experience to a user who has a large number of puffs in the first period and is estimated to have a preference of short-term concentration.

On the other hand, as indicated by the line 23, the controller 116 decreases the target temperature in the second period as the number of puffs indicated by the first puff information decreases. It is estimated that as the target temperature decreases, the amounts of aerosol and flavor component delivered to the user per puff decrease, and the life of the stick substrate 150 increases. In this regard, this configuration makes it possible to provide an optimal user experience to a user who has a small number of puffs in the first period and is estimated to have a preference of long-term stability.

As illustrated in Fig. 6, the controller 116 controls, based on the first puff information, the length of the second period. In particular, the controller 116 controls, based on the first puff information, the length of a period during which the second target temperature is maintained after the second target temperature is reached in the second period. The length of the period during which the second target temperature is maintained in the line 21 is expressed by 260 seconds - 180 seconds = 80 seconds. The length of the period during which the second target temperature is maintained in the line 22 is expressed by 200 seconds - 160 seconds = 40 seconds. The length of the period during which the second target temperature is maintained in the line 23 is expressed by 380 seconds - 260 seconds = 120 seconds. As a result of controlling the length of the second period, it is possible to provide a user experience that meets the preference of the user.

Specifically, as indicated by the line 22, the controller 116 decreases the length of the second period as the number of puffs indicated by the first puff information increases. It is estimated that as the number of puffs taken in the first period increases, the amount of the aerosol source remaining in the stick substrate 150 at the start of the second period decreases, and the life of the stick substrate 150 decreases. In this regard, this configuration makes it possible to generate an aerosol in a short period until the aerosol source is exhausted while preventing heating from being continued even after the aerosol source is exhausted. Accordingly, it is possible to improve the quality of user experience provided to a user who is estimated to have a preference of short-term concentration.

On the other hand, as indicated by the line 23, the controller 116 increases the length of the second period as the number of puffs indicated by the first puff information decreases. It is estimated that as the number of puffs taken in the first period decreases, the amount of the aerosol source remaining in the stick substrate 150 at the start of the second period increases, and the life of the stick substrate 150 increases. In this regard, this configuration makes it possible to generate an aerosol in a long period while preventing heating from being ended while a large amount of the aerosol source remains. Accordingly, it is possible to improve the quality of user experience provided to a user who is estimated to have a preference of long-term stability.

Two or more of the control of the temperature rising speed in the second period, the control of the target temperature in the second period, and the control of the length of the second period described above may be performed in combination.

These control operations may be executed based on other control contents as well as the first puff information.

In an example, the controller 116 may control, based on the temperature rising speed in the second period, the length of the second period. Specifically, the controller 116 may decrease the length of the second period as the temperature rising speed in the second period increases, and may increase the length of the second period as the temperature rising speed in the second period decreases.

In another example, the controller 116 may control, based on the length of the second period, the target temperature in the second period. Specifically, the controller 116 may decrease the target temperature in the second period as the length of the second period increases, and may increase the target temperature in the second period as the length of the second period decreases.

### (4) Flow of process

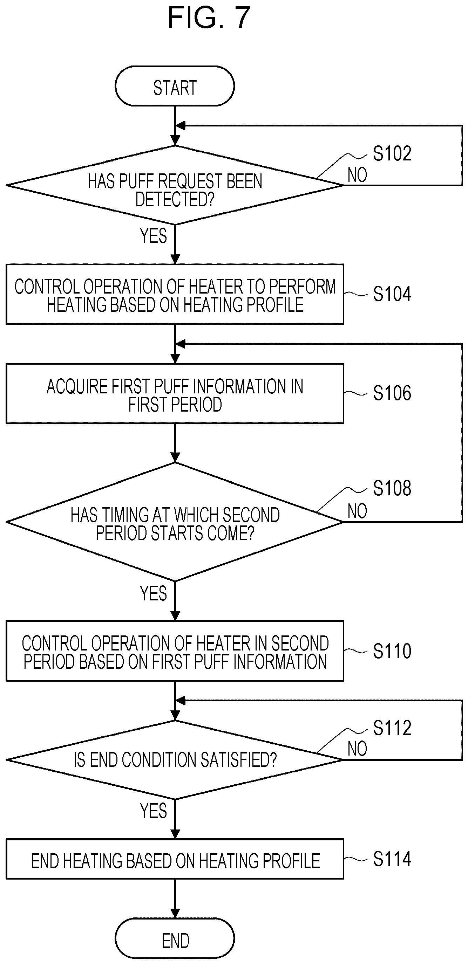

Fig. 7 is a flowchart illustrating an example of a flow of a process executed by the inhaler device 100 according to the present embodiment.

As illustrated in Fig. 7, first, the controller 116 determines whether a puff request has been detected (step S102). The puff request is a user operation requesting generation of an aerosol. An example of the puff request is an operation on the inhaler device 100, such as an operation on a switch or the like provided in the inhaler device 100. Another example of the puff request is insertion of the stick substrate 150 into the inhaler device 100. The insertion of the stick substrate 150 into the inhaler device 100 may be detected by a capacitive proximity sensor that detects the capacitance in a space near the opening 142, a pressure sensor that detects the pressure in the internal space 141, or the like.

If it is determined that a puff request has not been detected (NO in step S102), the controller 116 waits until a puff request has been detected.

On the other hand, if it is determined that a puff request has been detected (YES in step S102), the controller 116 controls the operation of the heater 121 to perform heating based on a heating profile (step S104). For example, the controller 116 starts, based on the heating profile, supply of electric power from the power supply 111 to the heater 121.

Subsequently, the controller 116 acquires first puff information in a first period (step S106). For example, the controller 116 acquires, as first puff information, information indicating the number of puffs taken in the first period. The number of times a value generated in accordance with a puff is detected by a pressure sensor, a flow sensor, a temperature sensor, or the like may be acquired as information indicating the number of puffs.

Next, the controller 116 determines whether the timing at which a second period starts has come (step S108). For example, the controller 116 determines the timing at which the intermediate temperature drop period ends as the timing at which the second period starts.

If it is determined that the timing at which the second period starts has not come (NO in step S108), the process returns to step S106.

If it is determined that the timing at which the second period starts has come (YES in step S108), the controller 116 controls, based on the first puff information, the operation of the heater 121 in the second period (step S11 0). For example, the controller 116 controls, based on the first puff information, at least any one of the temperature rising speed of the heater 121 in the second period, the second target temperature, or the length of the period during which the second target temperature is maintained.

Subsequently, the controller 116 determines whether an end condition is satisfied (step S112). An example of the end condition is that the period during which the second target temperature is maintained, the length of which is controlled based on the first puff information, has ended. Another example of the end condition is that the number of puffs from the start of heating has reached a predetermined number

If it is determined that the end condition is not satisfied (NO in step S112), the controller 116 waits until the end condition is satisfied.

If it is determined that the end condition is satisfied (YES in step S112), the controller 116 ends the heating based on the heating profile (step S114). Specifically, the controller 116 ends supply of electric power from the power supply 111 to the heater 121. Thereafter, the process ends.

### <3. Modifications>

### (1) First modification

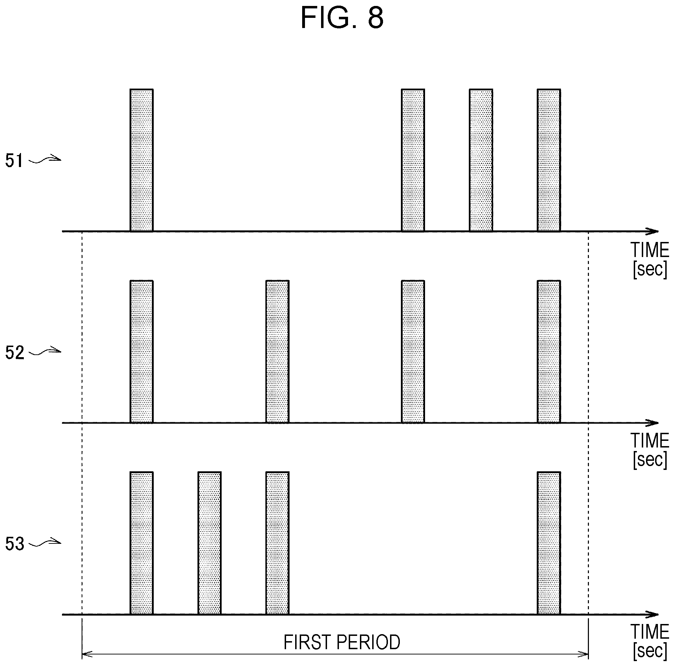

The first puff information may include information indicating a change in the puff interval in the first period. The information indicating a change in the puff interval in the first period may be information indicating a tendency of a time-series change in the puff interval in the first period. The first puff information according to the present modification will be described in detail with reference to Fig. 8.

Fig. 8 includes graphs each showing an example of puffs taken in the first period. In graphs 51 to 53, the horizontal axis represents time, and a rectangle represents a timing at which a puff is taken. The graph 51 shows an example in which the puff interval becomes shorter with elapse of time in the first period. The graph 52 shows an example in which the puff interval is constant regardless of elapse of time in the first period. The graph 53 shows an example in which the puff interval becomes longer with elapse of time in the first period. As shown in the graph 51, when the puff interval becomes shorter with elapse of time in the first period, it is estimated that the puff interval is short also in the second period subsequent to the first period. That is, it is estimated that the user having the tendency shown in the graph 51 has a preference of short-term concentration in the second period. As shown in the graph 53, when the puff interval becomes longer with elapse of time in the first period, it is estimated that the puff interval is long also in the second period subsequent to the first period. That is, it is estimated that the user having the tendency shown in the graph 53 has a preference of long-term stability in the second period.

Thus, the controller 116 performs control similar to the control described in the above embodiment in accordance with the user preference estimated from the first puff information.

That is, the controller 116 controls, based on the first puff information, the temperature rising speed of the heater 121 in the second period. In particular, the controller 116 controls, based on the first puff information, the temperature rising speed from when the second period starts to when the second target temperature is reached. This configuration makes it possible to provide a user experience that meets the preference of the user

Specifically, as indicated by the line 22 in Fig. 6, when the first puff information indicates that the puff interval becomes shorter with elapse of time, the controller 116 increases the temperature rising speed of the heater 121 in the second period. This configuration makes it possible to improve the quality of user experience provided to a user who is estimated to have a preference of short-term concentration.

On the other hand, as indicated by the line 23 in Fig. 6, when the first puff information indicates that the puff interval becomes longer with elapse of time, the controller 116 decreases the temperature rising speed of the heater 121 in the second period. This configuration makes it possible to improve the quality of user experience provided to a user who is estimated to have a preference of long-term stability.

In addition, the controller 116 controls, based on the first puff information, the target temperature in the second period. In particular, the controller 116 controls the second target temperature based on the first puff information. This configuration makes it possible to provide a user experience that meets the preference of the user

Specifically, as indicated by the line 22 in Fig. 6, when the first puff information indicates that the puff interval becomes shorter with elapse of time, the controller 116 increases the target temperature in the second period. This configuration makes it possible to improve the quality of user experience provided to a user who is estimated to have a preference of short-term concentration.

On the other hand, as indicated by the line 23 in Fig. 6, when the first puff information indicates that the puff interval becomes longer with elapse of time, the controller 116 decreases the target temperature in the second period. This configuration makes it possible to improve the quality of user experience provided to a user who is estimated to have a preference of long-term stability.

In addition, the controller 116 controls, based on the first puff information, the length of the second period. In particular, the controller 116 controls, based on the first puff information, the length of a period during which the second target temperature is maintained after the second target temperature is reached in the second period. This configuration makes it possible to provide a user experience that meets the preference of the user.

Specifically, as indicated by the line 22 in Fig. 6, when the first puff information indicates that the puff interval becomes shorter with elapse of time, the controller 116 decreases the length of the second period. This configuration makes it possible to improve the quality of user experience provided to a user who is estimated to have a preference of short-term concentration.

On the other hand, as indicated by the line 23 in Fig. 6, when the first puff information indicates that the puff interval becomes longer with elapse of time, the controller 116 increases the length of the second period. This configuration makes it possible to improve the quality of user experience provided to a user who is estimated to have a preference of long-term stability.

### (2) Second modification

In the above-described embodiment, a description has been given of an example in which the operation of the heater 121 in the second period is controlled based on the first puff information regarding a puff taken in the first period, but the present invention is not limited to such an example. The controller 116 may control the operation of the heater 121 in the second period, based on second puff information regarding a puff of an aerosol taken by a user in the second period. Specifically, the controller 116 may acquire second puff information regarding a puff of an aerosol taken by a user until a predetermined time elapses from the start of the second period, and may control the operation of the heater 121 in the period after the predetermined time elapses in the second period. Also with this configuration, effects similar to those of the above-described embodiment and the first modification are obtained.

The second puff information includes at least either one of information indicating the number of puffs taken in the second period or information indicating a change in the puff interval in the second period. The information indicating the number of puffs taken in the second period may be the number of puffs taken until a predetermined time elapses from the start of the second period, or may be the number of puffs per unit time. The information indicating a change in the puff interval in the second period may be information indicating the tendency of a time-series change in the puff interval until a predetermined time elapses from the start of the second period.

The controller 116 performs control similar to the control described in the above-described embodiment or the above-described first modification in accordance with a user preference estimated from the second puff information.

That is, the controller 116 controls, based on the second puff information, the temperature rising speed of the heater 121 in the second period. In particular, the controller 116 controls, based on the second puff information, the temperature rising speed from when the second period starts to when the second target temperature is reached. This configuration makes it possible to provide a user experience that meets the preference of the user.

Specifically, as described in the above embodiment, the controller 116 increases the temperature rising speed of the heater 121 in the second period as the number of puffs indicated by the second puff information increases. Alternatively, as described in the above first modification, the controller 116 increases the temperature rising speed of the heater 121 in the second period when the second puff information indicates that the puff interval becomes shorter with elapse of time. This configuration makes it possible to improve the quality of user experience provided to a user who is estimated to have a preference of short-term concentration.

On the other hand, as described in the above embodiment, the controller 116 decreases the temperature rising speed of the heater 121 in the second period as the number of puffs indicated by the second puff information decreases. Alternatively, as described in the above first modification, the controller 116 decreases the temperature rising speed of the heater 121 in the second period when the second puff information indicates that the puff interval becomes longer with elapse of time. This configuration makes it possible to improve the quality of user experience provided to a user who is estimated to have a preference of long-term stability.

In addition, the controller 116 controls, based on the second puff information, the target temperature in the second period. In particular, the controller 116 controls the second target temperature based on the second puff information. This configuration makes it possible to provide a user experience that meets the preference of the user

Specifically, as described in the above embodiment, the controller 116 increases the target temperature in the second period as the number of puffs indicated by the second puff information increases. Alternatively, as described in the above first modification, the controller 116 increases the target temperature in the second period when the second puff information indicates that the puff interval becomes shorter with elapse of time. This configuration makes it possible to improve the quality of user experience provided to a user who is estimated to have a preference of short-term concentration.

On the other hand, as described in the above embodiment, the controller 116 decreases the target temperature in the second period as the number of puffs indicated by the second puff information decreases. Alternatively, as described in the above first modification, the controller 116 decreases the target temperature in the second period when the second puff information indicates that the puff interval becomes longer with elapse of time. This configuration makes it possible to improve the quality of user experience provided to a user who is estimated to have a preference of long-term stability.

In addition, the controller 116 controls, based on the second puff information, the length of the second period. In particular, the controller 116 controls, based on the second puff information, the length of a period during which the second target temperature is maintained after the second target temperature is reached in the second period. This configuration makes it possible to provide a user experience that meets the preference of the user.

Specifically, as described in the above embodiment, the controller 116 decreases the length of the second period as the number of puffs indicated by the second puff information increases. Alternatively, as described in the above first modification, the controller 116 decreases the length of the second period when the second puff information indicates that the puff interval becomes shorter with elapse of time. This configuration makes it possible to improve the quality of user experience provided to a user who is estimated to have a preference of short-term concentration.

On the other hand, as described in the above embodiment, the controller 116 increases the length of the second period as the number of puffs indicated by the second puff information decreases. Alternatively, as described in the above first modification, the controller 116 increases the length of the second period when the second puff information indicates that the puff interval becomes longer with elapse of time. This configuration makes it possible to improve the quality of user experience provided to a user who is estimated to have a preference of long-term stability.

### <4. Supplementary description>

While a preferred embodiment of the present invention has been described in detail with reference to the accompanying drawings, the present invention is not limited to the foregoing examples. It will be apparent that those skilled in the art to which the present invention belongs are able to conceive of various modifications or variations within the scope of the technical ideas described in the claims, and it is understood that such modifications or variations also belong to the technical scope of the present invention.

For example, in the above-described embodiment, an example has been described in which the target temperature in the second period increases as the length of the second period decreases, and the target temperature in the second period decreases as the length of the second period increases, but the present invention is not limited to such an example. For example, the controller 116 may decrease the target temperature in the second period as the length of the second period decreases. It is estimated that as the number of puffs taken in the first period increases, the amount of the aerosol source remaining in the stick substrate 150 at the start of the second period decreases, and the life of the stick substrate 150 decreases. In this regard, this configuration makes it possible to extend the life of the stick substrate 150 by decreasing the target temperature. In addition, the controller 116 may increase the target temperature in the second period as the length of the second period increases. It is estimated that as the number of puffs taken in the first period decreases, the amount of the aerosol source remaining in the stick substrate 150 at the start of the second period increases, and the life of the stick substrate 150 increases. In this regard, this configuration makes it possible to prevent heating from being ended while a large amount of aerosol source remains, by increasing the target temperature.

For example, in the above-described embodiment, an example has been described in which the first puff information is information indicating the number of puffs taken in the first period, but the present invention is not limited to such an example. The first puff information may be information indicating the amount of aerosol inhaled by the user in the first period. As the amount of aerosol inhaled by the user increases, the user is more likely to have a preference of short-term concentration. On the other hand, as the amount of aerosol inhaled by the user decreases, the user is more likely to have a preference of long-term stability. The information indicating the amount of aerosol inhaled by the user in the first period is detected by, for example, a flow sensor. When it is difficult to detect the amount of aerosol inhaled by the user in the first period, the amount of air that has flowed into the internal space 141 or that has flowed out of the internal space 141 in the first period may be used as a substitute. The same applies to the second puff information.

A series of processes performed by the individual devices described in this specification may be implemented by using any of software, hardware, and a combination of software and hardware. Programs constituting the software are stored in advance in, for example, a recording medium (specifically, a non-transitory computer-readable storage medium) provided inside or outside each device. Each program is read into a RAM and is executed by a processor such as a CPU when being executed by a computer that controls each device described in this specification, for example. The recording medium is, for example, a magnetic disk, an optical disc, a magneto-optical disc, a flash memory, or the like. In addition, the foregoing computer programs may be distributed via a network, for example, without using a recording medium.

In addition, the process described using a flowchart and a sequence diagram in this specification need not necessarily be executed in the illustrated order. Some processing steps may be executed in parallel. In addition, an additional processing step may be employed, and some processing steps may be omitted.

The following configurations also belong to the technical scope of the present invention.
(1) An inhaler device including:
   a heater configured to heat a substrate including an aerosol source to generate an aerosol; and
   a controller configured to control, based on a temperature setting defining a time-series transition of a target temperature which is a target value of a temperature of the heater, an operation of the heater, wherein
   the controller is configured to control, based on first puff information regarding a puff of the aerosol taken by a user in a first period in a period during which the substrate which is a single substrate is heated based on the temperature setting, an operation of the heater in a second period subsequent to the first period.
(2) The inhaler device according to (1) above, wherein
   the first puff information includes information indicating the number of puffs taken in the first period.
(3) The inhaler device according to (2) above, wherein
   the controller is configured to control, based on the first puff information, a temperature rising speed of the heater in the second period.
(4) The inhaler device according to (3) above, wherein
   the controller is configured to increase the temperature rising speed of the heater in the second period as the number of puffs indicated by the first puff information increases, and decrease the temperature rising speed of the heater in the second period as the number of puffs indicated by the first puff information decreases.
(5) The inhaler device according to any one of (2) to (4) above, wherein
   the controller is configured to control, based on the first puff information, the target temperature in the second period.
(6) The inhaler device according to (5) above, wherein
   the controller is configured to increase the target temperature in the second period as the number of puffs indicated by the first puff information increases, and decrease the target temperature in the second period as the number of puffs indicated by the first puff information decreases.
(7) The inhaler device according to any one of (2) to (6) above, wherein
   the controller is configured to control, based on the first puff information, a length of the second period.
(8)The inhaler device according to any one of (2) to (7) above, wherein
   the controller is configured to decrease a length of the second period as the number of puffs indicated by the first puff information increases, and increase the length of the second period as the number of puffs indicated by the first puff information decreases.
(9) The inhaler device according to any one of (1) to (8) above, wherein
   the first puff information includes information indicating a change in a puff interval in the first period.
(10) The inhaler device according to (9) above, wherein
   the controller is configured to increase a temperature rising speed of the heater in the second period when the first puff information indicates that the puff interval becomes shorter with elapse of time, and decrease the temperature rising speed of the heater in the second period when the first puff information indicates that the puff interval becomes longer with elapse of time.
(11) The inhaler device according to (9) or (10) above, wherein
   the controller is configured to increase the target temperature in the second period when the first puff information indicates that the puff interval becomes shorter with elapse of time, and decrease the target temperature in the second period when the first puff information indicates that the puff interval becomes longer with elapse of time.
(12) The inhaler device according to any one of (9) to (11) above, wherein
   the controller is configured to decrease a length of the second period when the first puff information indicates that the puff interval becomes shorter with elapse of time, and increase the length of the second period when the first puff information indicates that the puff interval becomes longer with elapse of time.
(13) The inhaler device according to any one of (1) to (12) above, wherein
   the controller is configured to control, based on second puff information regarding a puff of the aerosol taken by the user in the second period, the operation of the heater in the second period.
(14) The inhaler device according to (13) above, wherein
   the second puff information includes at least either one of information indicating the number of puffs taken in the second period or information indicating a change in a puff interval in the second period.
(15) The inhaler device according to (13) or (14) above, wherein
   the controller is configured to control, based on the second puff information, a temperature rising speed of the heater in the second period.
(16) The inhaler device according to any one of (13) to (15) above, wherein
   the controller is configured to control, based on the second puff information, the target temperature in the second period.
(17) The inhaler device according to any one of (13) to (16) above, wherein
   the controller is configured to control, based on the second puff information, a length of the second period.
(18) The inhaler device according to any one of (1) to (17) above, wherein
   the temperature setting includes
      an initial temperature rise period during which the temperature of the heater rises from an initial temperature,
      an intermediate temperature drop period during which the temperature of the heater drops after the initial temperature rise period, and
      a temperature re-rise period during which the temperature of the heater rises after the intermediate temperature drop period,
   the first period is included in at least either one of the initial temperature rise period or the intermediate temperature drop period, and
   the second period is included in the temperature re-rise period.
(19) A substrate that is to be heated by an inhaler device to generate an aerosol and that includes an aerosol source, the inhaler device including:
   a heater configured to heat the substrate including the aerosol source to generate an aerosol; and
   a controller configured to control, based on a temperature setting defining a time-series transition of a target temperature which is a target value of a temperature of the heater, an operation of the heater, wherein
   the controller is configured to control, based on first puff information regarding a puff of the aerosol taken by a user in a first period in a period during which the substrate which is a single substrate is heated based on the temperature setting, an operation of the heater in a second period subsequent to the first period.
(20) A control method for controlling an inhaler device including a heater configured to heat a substrate including an aerosol source to generate an aerosol, the control method including:
   controlling, based on a temperature setting defining a time-series transition of a target temperature which is a target value of a temperature of the heater, an operation of the heater, wherein
   the controlling of an operation of the heater includes controlling, based on first puff information regarding a puff of the aerosol taken by a user in a first period in a period during which the substrate which is a single substrate is heated based on the temperature setting, an operation of the heater in a second period subsequent to the first period.

### Reference Signs List

- 100: inhaler device
- 111: power supply
- 112: sensor
- 113: notifier
- 114: memory
- 115: communicator
- 116: controller
- 121: heater
- 140: holder
- 141: internal space
- 142: opening
- 143: bottom
- 150: stick substrate
- 151: substrate
- 152: inhalation port

## Claims

1. An inhaler device comprising:
a heater configured to heat a substrate including an aerosol source to generate an aerosol; and
a controller configured to control, based on a temperature setting defining a time-series transition of a target temperature which is a target value of a temperature of the heater, an operation of the heater, wherein
the controller is configured to control, based on first puff information regarding a puff of the aerosol taken by a user in a first period in a period during which the substrate which is a single substrate is heated based on the temperature setting, an operation of the heater in a second period subsequent to the first period.

2. The inhaler device according to claim 1, wherein
the first puff information includes information indicating the number of puffs taken in the first period.

3. The inhaler device according to claim 2, wherein
the controller is configured to control, based on the first puff information, a temperature rising speed of the heater in the second period.

4. The inhaler device according to claim 3, wherein
the controller is configured to increase the temperature rising speed of the heater in the second period as the number of puffs indicated by the first puff information increases, and decrease the temperature rising speed of the heater in the second period as the number of puffs indicated by the first puff information decreases.

5. The inhaler device according to any one of claims 2 to 4, wherein
the controller is configured to control, based on the first puff information, the target temperature in the second period.

6. The inhaler device according to claim 5, wherein
the controller is configured to increase the target temperature in the second period as the number of puffs indicated by the first puff information increases, and decrease the target temperature in the second period as the number of puffs indicated by the first puff information decreases.

7. The inhaler device according to any one of claims 2 to 6, wherein
the controller is configured to control, based on the first puff information, a length of the second period.

8. The inhaler device according to any one of claims 2 to 7, wherein
the controller is configured to decrease a length of the second period as the number of puffs indicated by the first puff information increases, and increase the length of the second period as the number of puffs indicated by the first puff information decreases.

9. The inhaler device according to any one of claims 1 to 8, wherein
the first puff information includes information indicating a change in a puff interval in the first period.

10. The inhaler device according to claim 9, wherein
the controller is configured to increase a temperature rising speed of the heater in the second period when the first puff information indicates that the puff interval becomes shorter with elapse of time, and decrease the temperature rising speed of the heater in the second period when the first puff information indicates that the puff interval becomes longer with elapse of time.

11. The inhaler device according to claim 9 or 10, wherein
the controller is configured to increase the target temperature in the second period when the first puff information indicates that the puff interval becomes shorter with elapse of time, and decrease the target temperature in the second period when the first puff information indicates that the puff interval becomes longer with elapse of time.

12. The inhaler device according to any one of claims 9 to 11, wherein
the controller is configured to decrease a length of the second period when the first puff information indicates that the puff interval becomes shorter with elapse of time, and increase the length of the second period when the first puff information indicates that the puff interval becomes longer with elapse of time.

13. The inhaler device according to any one of claims 1 to 12, wherein
the controller is configured to control, based on second puff information regarding a puff of the aerosol taken by the user in the second period, the operation of the heater in the second period.

14. The inhaler device according to claim 13, wherein
the second puff information includes at least either one of information indicating the number of puffs taken in the second period or information indicating a change in a puff interval in the second period.

15. The inhaler device according to claim 13 or 14, wherein
the controller is configured to control, based on the second puff information, a temperature rising speed of the heater in the second period.

16. The inhaler device according to any one of claims 13 to 15, wherein
the controller is configured to control, based on the second puff information, the target temperature in the second period.

17. The inhaler device according to any one of claims 13 to 16, wherein
the controller is configured to control, based on the second puff information, a length of the second period.

18. The inhaler device according to any one of claims 1 to 17, wherein
the temperature setting includes
an initial temperature rise period during which the temperature of the heater rises from an initial temperature, an intermediate temperature drop period during which the temperature of the heater drops after the initial temperature rise period, and
a temperature re-rise period during which the temperature of the heater rises after the intermediate temperature drop period,
the first period is included in at least either one of the initial temperature rise period or the intermediate temperature drop period, and
the second period is included in the temperature re-rise period.

19. A substrate that is to be heated by an inhaler device to generate an aerosol and that includes an aerosol source, the inhaler device comprising:
a heater configured to heat the substrate including the aerosol source to generate an aerosol; and
a controller configured to control, based on a temperature setting defining a time-series transition of a target temperature which is a target value of a temperature of the heater, an operation of the heater, wherein
the controller is configured to control, based on first puff information regarding a puff of the aerosol taken by a user in a first period in a period during which the substrate which is a single substrate is heated based on the temperature setting, an operation of the heater in a second period subsequent to the first period.

20. A control method for controlling an inhaler device including a heater configured to heat a substrate including an aerosol source to generate an aerosol, the control method comprising:
controlling, based on a temperature setting defining a time-series transition of a target temperature which is a target value of a temperature of the heater, an operation of the heater, wherein
the controlling of an operation of the heater includes controlling, based on first puff information regarding a puff of the aerosol taken by a user in a first period in a period during which the substrate which is a single substrate is heated based on the temperature setting, an operation of the heater in a second period subsequent to the first period.
